# EUROPEAN PATENT SPECIFICATION

(11) **EP 1 155 114 B1**
(45) Date of publication and mention of the grant of the patent: **14.11.2007**
(21) Application number: 00904108.8
(22) Date of filing: 11.02.2000
(51) Int. Cl.: C12N 5/00

(54) **AVIAN PLURIPOTENT EMBRYONIC GERM CELL LINE**
PLURIPOTENTE EMBRYONALE KEIMZELLINIE AUS VÖGELN
LIGNEE DE CELLULES GERMINALES EMBRYONNAIRES MULTIPOTENTES AVIAIRES

(30) Priority: 11.02.1999 KR 9904860
(43) Date of publication of application: 21.11.2001
(73) Proprietor: Hanmi Pharm. Co., Ltd., Kyungki-do 445-910 (KR); Han, Jae Yong, Yongin-city, Kyonggi-do 449-840 (KR)
(72) Inventor: HAN, Jae Yong, Yongin-city, Kyonggi-do 449-840 (KR); PARK, Tae Sub, Anyang-shi, Kyonggi-do 431-058 (KR)
(74) Representative: Lorenz, Werner
(86) International application number: PCT/KR2000/000104
(87) International publication number: WO 2000/047717

(56) References cited:
- WO-A-99/06534
- US-A- 5 340 740
- HAN J Y ET AL: "PRIMORDIAL GERM CELLS IN AVES - REVIEW -" ASIAN-AUSTRALASIAN JOURNAL OF ANIMAL SCIENCES, SUWEON, KR, vol. 7, no. 4, December 1994 (1994-12), pages 459-466, XP002913030 ISSN: 1011-2367
- PONCE DE LEON F A ET AL: "RECENT ADVANCES IN CHICKEN PRIMORDIAL GERM CELL TECHNOLOGY" REVISTA BRASILEIRA DE REPRODUCAO ANIMAL, BELO HORIZONTE, BR, vol. 21, no. 3, 1997, pages 96-101, XP002913042 ISSN: 0102-0803
- TAE SUB PARK ET AL: "DERIVATION AND CHARACTERIZATION OF PLURIPOTENT EMBRYONIC GERM CELLSIN CHICKEN" MOLECULAR REPRODUCTION AND DEVELOPMENT, LISSS, NEW YORK, NY, US, vol. 56, August 2000 (2000-08), pages 475-482, XP000994678 ISSN: 1040-452X
- CELL BIOL. INT., vol. 21, no. 8, August 1997, pages 495 - 499
- TRANSGENIC RES., vol. 7, no. 4, July 1998, pages 247 - 252
- MOL. REPROD. DEV., vol. 39, no. 2, October 1994, pages 153 - 161
- CELL BIOL. INT., vol. 19, no. 7, July 1995, pages 569 - 576
- PROC. NATL. ACAD. SCI. USA, vol. 95, no. 23, November 1998, pages 13726 - 13731
- NATURE, vol. 353, October 1991, pages 750 - 752
- CELL, vol. 70, September 1992, pages 841 - 847
- J. EXP. ZOOL, vol. 280, no. 3, February 1998, pages 265 - 267
- BIOL. REPROD., vol. 58, no. 5, May 1998, pages 1321 - 1329
- BIOL. REPROD., vol. 59, no. 5, November 1998, pages 1224 - 1229

## Description

### FIELD OF THE INVENTION

The present invention relates to a process for preparing an established avian pluripotent embryonic germ cell line and an avian pluripotent embryonic germ cell line.

### BACKGROUND OF THE INVENTION

Embryonic stem (ES) cell lines are undifferentiated and pluripotent cells isolated from blastocyst or morula embryos. Although ES cells are expected to be highly useful, e.g., in the study of developmental biology, analysis of the characteristics of totipotent cells, and gene-targeting to produce genetically modified livestock, only mouse ES cells with proven germ-line transmission have been established (Evans, M. J. and Kaufman, M. H., Nature, 292, 154-156(1981); Bradley et al., Nature, 309, 255-256(1984)) and the use of ES cells in producing livestock has not yet become a reality because of limitations in germ-line transmission in species other than mice (First, N. L. et al., Reprod. Feril. Dev., 6, 553-562(1994); wheeler, M. B., Reprod. Fertil. Dev., 6, 563-568(1994); Giles, J. R. et al., Mol. Reprod. Dev., 36, 130-138(1993); and Doetschman, T. C. et al., Dev. Biol., 127, 224-227(1988)).

Recently, primordial germ cells (PGCs), which are the progenitors of sperm or egg cells that develop after sexual maturity, have been provided as an alternative source of pluripotent stem cells. The stem cells derived from PGCs are called embryonic germ (EG) cells (Resnick, J. L. et al., Nature, 359, 550-551(1992); and Matsui, Y. et al., Cell, 70, 841-847(1992)). Mouse PGCs have been successfully co-cultured on mitotically inactivated STO cells supplemented with three critical growth factors: stem cell factor (SCF), leukemia inhibitory factor (LIF), and basic fibroblast growth factor (bFGF) (Godin, J. R. et al., Mol. Reprod. Dev., 36, 130-138(1991); Dolci, S. et al., Nature, 352, 809-811(1991); Matsui, Y. et al., Nature, 353, 750-752(1991); and Resnick, J. L. et al., Nature, 359, 550-551(1992)). Resnick et al. reported that mouse PGCs continued to proliferate after subculture and formed colonies of cells that resembled ES cells (Resnick, J. L. et al., Nature, 359, 550-551(1992)). Labosky et al. demonstrated that murine EG cell lines had the pluripotency needed for germ-line transmission in vivo (Labosky, P. A. et al., Development, 120, 3197-3204(1994)).

As to bovine and porcine EG cells, there have been characterized traits such as morphology, alkaline phosphatase activity, and embryoid body formation (Cherny, R. A. et al., Reprod. Fertil. Dev., 6, 569-575(1994); Shim, H. et al., Biol. Reprod., 57, 1189-1095(1997); Piedrahita, J. A. et al., J. Reprod. Fertil., 52, 245-254(1997)). However, germ-line transmission has not been proven in these species.

Pain et al. reported that avian stem cells with multiple morphogenetic potentialities were derived and maintained in vitro by long-term culture of blastodermal cells. However, the production of pluripotent EG cells derived from PGCs has not previously been reported in any non-mammalian species.

In avian species, PGCs first arise from the epiblast and migrate to the hypoblast of the area pellucida (the germinal crescent) at stage 4, approximately 18-19 hours after incubation (Swift, C. H., Am. J. Anat., 15, 483-516(1914); Hamburger, V. and Hamilton, H. L., J. Morphol., 88, 49-92(1951); and Eyal-Giladi, H. and Kochav, S., Dev. Biol., 49, 321-337(1976)). PGCs move from the germinal crescent into the blood stream at stage 10-12 (Ando, Y. and Fujimoto, T., Dev. Growh Differ., 25, 345-32(1983); and Ukeshima, A. et al., J. Electron. Microsc., 40, 124-128(1991)) and circulate in the vascular system until stage 17 (2.5 days of incubation) when they reach the region of the germinal ridges, in which they finally concentrate and colonize (Nieuwkoop, P. D. and Sutasurya, L. A., In Primordial Germ Cells in the Chrodates, 113-127(1979)). This migration pathway and the subsequent developmental processes differ dramatically from the comparable processes in mammalian species.

Allioli et al. reported that chicken PGCs isolated from gonads could proliferate for several days under an in vitro culture condition (Allioli, N. et al., Dev. Biol., 165, 30-37(1994)). Chang et al. cultured chicken PGCs from gonads on stroma cells of the germinal ridge for 5 days (Chang, I. et al., Cell Biol. Int., 19, 569-676(1995)). These cultured gonadal PGCs had the ability to migrate to the germinal ridge when re-injected into recipient embryos. Recently, Chang et al. produced germ-line chimeric chickens by injection of gonadal PGCs which had been cultured in vitro for 5 days (Chang, I. et al., Cell Biol. Int., 21, 495-499(1997)).

Further, PCT publication No. WO 99/06534 discloses a method for establishing EG cells by culturing PGCs isolated from the blood of an embryo at stage 13 to 14 in a medium supplemented with LIF, bFGF, SCF and IGF-I without using a feeder layer. However, the amount of blood PGCs obtainable from an embryo, i.e., about 100 cells/embryo, is much less than that of gPGCs, i.e., more than 1000 cells/embryo, and it is difficult to isolate the blood PGCs from the embryonic blood. Further, although the PGCs were cultured without using a feeder layer, they attached to the wall of the culture vessel and morphologically changed only after 3 or 4 passages. Accordingly, it is presumed that the EG cells established therefrom would be already differentiated. Moreover, the EG cells were examined only on their alkaline phosphatase activity for the demonstration of the pluripotency thereof without examination on other properties and, accordingly, it is not certain whether the EG cells are indeed established. In addition, the reference did not teach or anticipate whether the EG cells have the in vitro and in vivo differentiating abilities, which are characteristics of an established EG cells, and whether they proliferate continuously after several passages.

Thus, there has continued to exist a need to develop an EG cell line transmitting foreign gene through multiple generations, thereby enabling the production of a transgenic livestock using foreign gene transfection and gene targeting.

### SUMMARY OF THE INVENTION

Accordingly, it is an object of the present invention to provide a process for establishing an avian pluripotent embryonic germ (EG) cell line.

Another object of the present invention is to provide an avian pluripotent embryonic germ cell line.

In accordance with one aspect of the present invention, there is provided a process for preparing an established avian embryonic germ cell line comprising the steps of:
(a) culturing primordial germ cells (PGCs) isolated from an avian embryonic gonad in a medium supplemented with a cell growth factor and a differentiation inhibitory factor to obtain EG cell colonies;
(b) culturing the EG cells in the same medium as in step (a) by employing a feeder layer until the EG cells are colonized; and
(c) recovering and subculturing the EG cells in the same medium as in step (a) to establish the EG cell line.

In accordance with another aspect of the present invention, there is provided a chicken embryonic germ cell line having characteristics substantially identical to that deposited under the accession number of KCLRF-BP-00026.

### BRIEF DESCRIPTION OF THE DRAWING

The above and other objects and features of the present invention will become apparent from the following description of the invention, when taken in conjunction with the accompanying drawing, in which:
Figs. 1a and 1b show the morphological characteristics of chicken EG cell colonies after 3 passages on chicken embryonic fibroblast (Fig. 1a : scale, 50 µm; and Fig. 1b : scale, 25 µm);
Fig. 2 presents the PAS reactivity in EG cell colonies formed after 4 passages;
Fig. 3 presents the anti-SSEA-1 antibody screening results in EG cell colonies formed after 4 passages;
Fig. 4 presents the proliferation assay result in EG cell colonies stained after 8 passages;
Fig. 5a displays the embryoid bodies formed from a chicken EG cells in suspension culture after 8 days; and Figs. 5b to 5d, the results of immunohistochemical analyses of the embryoid bodies using antibodies against actin, α-1-fectoprotein and S100, respectively;
Fig. 6a exhibits typical Korean Ogol chickens having black feather; and Fig. 6b shows a somatic chimera chicken having white patches around the neck and on the breast; and
Fig. 7 depicts the result of PCR analysis for somatic and germline chimerisms.

### DETAILED DESCRIPTION OF THE INVENTION

The process of the present invention for establishing an avian embryonic germ (EG) cell line begins with the isolation of avian primordial germ cells (PGCs). The avian may be turkey, chicken, quail, pheasant or duck. The PGCs may be isolated from the embryonic gonad of an early-stage avian embryo, at stage 14 to 36 (50 hours to 10 days after incubation), preferably, stage 24 to 30 (4 to 6.5 days after incubation). For instance, the embryonic gonad of White Leghorn at stage 28 is isolated and then the gonad tissue is dissociated in trypsin-EDTA to obtain a suspension containing gonadal primordial germ cells (gPGC). However, the developmental stage of the embryo is not limitative unless it impairs the purpose of the present invention, and may vary according to the avian species or the kind of organ, tissue and membrane from which the PGCs are separated.

The isolated PGCs are cultured at a temperature ranging from 37 °C to 42 °C under an atmosphere containing 5% CO₂ in a medium containing cell growth factors and a differentiation inhibitory factor until the colonization of EG cells. The medium is preferably DMEM (Dulbecco's Modified Eagle's Medium; Gibco BRL cat#, 10313-021) or a functional equivalent thereof.

Exemplary cell growth factors useful in the present invention include stem cell factor (SCF), basic fibroblast growth factor (bFGF), interleukin-11 (IL-11), insulin-like growth factor-I (IGF-I) and a mixture thereof. Representative differentiation inhibitory factor is leukemia inhibitory factor (LIF). The culture medium may further contain mammalian serum, avian serum or a supplementary ingredient selected from the group consisting of sodium pyruvate, glutamine, β-mercaptoethanol and a mixture thereof.

The culture medium is preferably DMEM supplemented with 0.1 to 30% fetal bovine serum (FBS), 0.02 to 20 % chicken serum, 0.01 to 100 mM sodium pyruvate, 0.02 to 200 mM glutamine, 0.55 to 5500 µM β-mercaptoethanol, 0.05 to 500 ng/ml of SCF, 0.1 to 1000 units/ml of LIF, 0.1 to 1000 ng/ml of bFGF, 0.0004 to 4 ng/ml of IL-11 and 0.1 to 1000 ng/ml of IGF-1.

The EG cell colonies formed on the medium is separated into individual EG cell, e.g., by repeated pipetting, and the EG cells are recovered, suspended in a medium, e.g., the medium described above, and then cultured at 37 to 42 °C for 7 to 10 days on a feeder layer until the colonization of cells exhibiting the morphological characteristics of an EG cell line. As the feeder layer, avian embryonic fibroblast or an equivalent thereof may be employed, while mitotically active avian embryonic fibroblast (CEF) or avian fibroblast is most preferred.

The resulting EG cells are passaged at an interval of 7 to 10 days in the same medium as above to establish an embryonic germ cell line. The embryonic germ cell line thus established may be maintained for a period of over 4 months by repeated subculture.

The present invention also provides a chicken EG cell line produced by using the inventive process.

The morphology of the chicken EG cell line colonies is multi-layered and the colonies are well-delineated. Each chicken EG cell is composed of a large nucleus and a relatively small amount of cytoplasm, similar to the separated morphologies of murine and porcine ES cells and EG cells (Wobus, A. M. et al., Exp. Cell. Res., 152, 212-2199(1984); Matsui, Y. et al., Cell, 70, 841-847(1992); Resnick, J. L. et al., Nature, 359, 550-551(1992); Shim, H. et al., Biol Reprod, 57, 1089-1095(1997); and, Piedrahita, J. A. et al., Biol. Reprod., 58, 1321-1329(1998)). However, the morphology of the chicken EG cells is slightly different from that of mouse ES or EG cells in that almost all of the colonies are uniformly round and that the prominent dark nucleoli observed in other species are not clearly discernible. Avian species are different from mammalian species in terms of physiology, development and differentiation of germ cells and, thus, the chicken EG cells are different from mouse EG cells in their shape, their adhesive properties, and other characteristics.

The chicken EG cells maintain the characteristics of gonadal PGCs and undifferentiated stem cells. The chicken EG cells express SSEA-1 antigen and, in an in vitro suspension culture, successfully develop into embryoid bodies which differentiate into a variety of cell types. Further, the chicken EG cells are confirmed to proliferate and differentiate into various tissues including the gonad during embryo development in an experiment using chickens and, thus, they are pluripotent in vivo.

One of the chicken embryonic germ cell lines established in the present invention was deposited on September 22, 1999 with the Korean Cell Line Research Foundation(KCLRF) (Address: Cancer Research Institute, Seoul National University College of Medicine, #28, Yongon-dong, Chongno-gu, Seoul, 110-744, Republic of Korea) under the terms of the Budapest Treaty on the International Recognition of the Deposit of Microorganism for the Purpose of Patent Procedure under the accession number of KCLRF-BP-00026.

A somatic or germline chimera can be produced by microinjection of EG cells into an egg, preferably, into the germinal cavity or blood vessel thereof. More preferably, the EG cell may be microinjected into the germinal cavity of an egg at stage X or into the blood vessel of an egg at stage ranging from 13 to 17.

A desired foreign gene can be transfected and introduced in the inventive chicken EG cells by electroporation or liposome, and stably-transfected chicken EG cells can be selected by passaging them in a medium containing an antibiotic.

Thus, the inventive chicken EG cell lines are useful for the production of transgenic chickens and for studies of germ cell differentiation and genomic imprinting.

The following Examples are intended to further illustrate the present invention without limiting its scope.

Further, percentages given below for solid in solid mixture, liquid in liquid, and solid in liquid are on a wt/wt, vol/vol and wt/vol basis, respectively, unless specifically indicated otherwise.

### Example 1 : Isolation of PGCs and establishment of culture condition for preparing a chicken EG cell line

### (Step 1) Isolation of PGCs

A fertilized egg of White Leghorn obtained from the College of Agriculture and Life Sciences, Seoul National University was incubated for 5.5 days (until stage 28) at 37.5 °C and a relative humidity of 60-70%. The embryo was extracted from the fertilized egg at stage 28 and washed in a 100 mm petri dish with magnesium-free phosphate buffered saline (PBS) to remove the yolk and blood. The embryo was transferred to a petri dish coated with a black wax and the embryonic gonads were isolated therefrom with forceps. The gonad tissue was separated into individual gonadal primordial germ cells (gPGCs) by treating with 0.25% trypsin-0.05% EDTA. Added thereto was DMEM (Dulbecco's Modified Eagle's Medium, Gibco BRL, USA) containing 10% FBS (fetal bovine serum, Gibco, USA) to inactivate trypsin-EDTA, and gPGCs were harvested by centrifugation.

### (Step 2) Establishment of culture condition

The EG (embryonic germ) cell culture medium (i.e., DMEM) was supplemented with one or more of growth factors selected from stem cell factor (SCF), leukemia inhibitory factor (LIF), and basic fibroblast growth factor (bFGF). SCF, LIF, and bFGF have been reported to be important growth factors for the survival and proliferation of PGCs in the mouse (Resnick, J. L. et al., Nature, 359, 550-551(1992); and Donovan, P. J., Curr. Top. Dev. Biol., 29, 189-225(1994)). In addition to these three factors, growth-related factors such as interleukin-11 (IL-11) and insulin-like growth factor-I (IGF-I) were added to the medium. The gPGCs obtained in Step 1 were suspended in the culture medium supplemented with various combinations of the above factors and then incubated at 37 °C under an atmosphere containing 5% CO₂ until the colonization of the EG cells.

Tests showed that colonization of EG cells does not occur in the absence of IL-11 and IGF-I. Therefore, IL-11 and IGF-I are essential for the survival and proliferation of chicken EG cells.

### Example 2 : Culture of chicken EG cells

The EG cells obtained in Example 1 were seeded in a 24-well culture plate containing EG cell culture media consisting of DMEM (Gibco, USA) supplemented with 10% FBS, 2% chicken serum (Gibco, USA), 1mM sodium pyruvate, 2mM L-glutamine, 5.5 x 10⁻⁵ M β-mercaptoethanol, 100 µg/ml of streptomycin, 100 units/ml of penicillin, 5 ng/ml of human stem cell factor (hSCF; Sigma, USA), 10 units/ml of murine leukemia inhibitory factor (mLIF; Sigma, USA), 10 ng/ml of bovine basic fibroblast growth factor (bFGF; Sigma, USA), 0.04 ng/ml of human interleukin-11 (h-IL-11; Sigma, USA) and 10 ng/ml of human insulin-like growth factor-I (IGF-I; Sigma, USA) and incubated in an incubator for 7-10 days at 37°C under an atmosphere of 5% CO₂ to produce EG cell colonies deposited on a layer of germinal ridge stroma cells (GRSC). The colonies of chicken EG cells were separated from the GRSC layer by gentle pipetting and harvested by centrifuge at 200 X g for 5 minutes. The harvested EG cells were suspended in DMEM and divided into a fresh 24-well plate together with chicken embryonic fibroblasts (CEFs) which were not mitotically inactivated. The EG cell colonies were passaged at an interval of 7 to 10 days under the condition as above. These colonies were maintained for up to 10 passages and proliferated over a period of 4 months in repeated subculture.

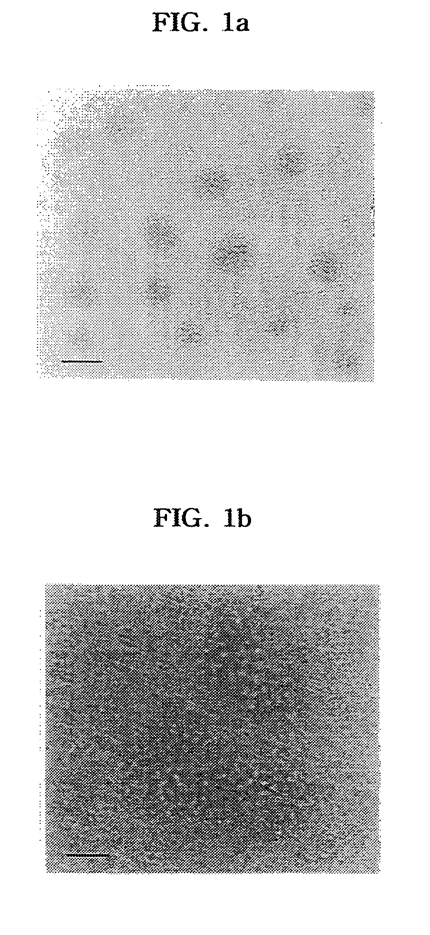

Figs. 1a and 1b show chicken EG cell colonies after 3 passages on chicken embryonic fibroblast (CEF) cells (Fig. 1a: scale, 50 µm; and Fig. 1b: scale, 25 µm). The morphology of the chicken EG cells was slightly different from that of mouse ES or EG cells. Almost all of the chicken EG cell colonies were uniformly round and were not tightly bound to the CEF feeder layer. In contrast to mouse ES or EG cells, chicken EG cells did not pack strongly together and; it was not difficult to discern the individual component cells. The morphology of the colonies was multi-layered and the boundaries thereof was well-delineated. The chicken EG cell was composed of a large nucleus and a relatively small amount of cytoplasm, while its nucleoli was not prominent.

### Example 3: Characterization of the EG cell

To determine whether the pluripotency of the EG cell possessed characteristic features of a pluripotent cell, the presence of glycogens and SSEA-1 epitope as well as its alkaline phosphatase activity and abilities to proliferate and differentiate in vitro were examined.

### (1) Periodic Acid-Shiff's (PAS) staining

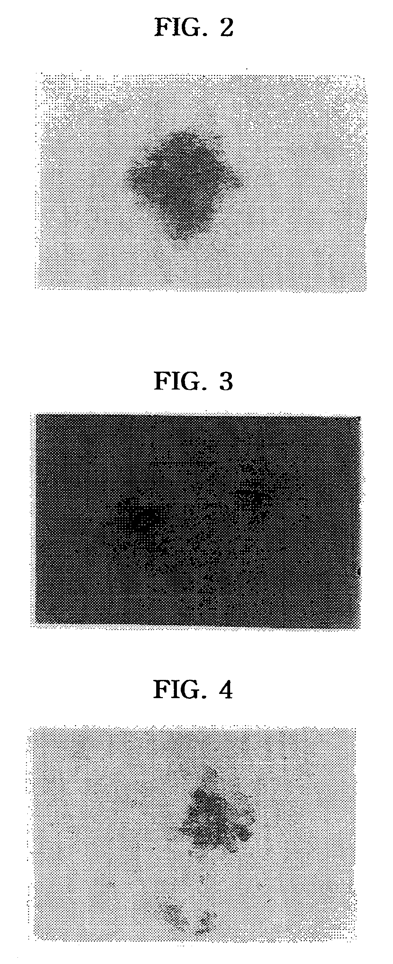

The EG cell colonies after 4 passages obtained in Example 2 were fixed to a plate in a 1 % glutaraldehyde solution for 5 min. and washed twice with an equal volume of phosphate buffered saline(PBS). The EG cell colonies were immersed in Periodic Acid Solution(Sigma, USA) at room temperature for 5 min. and then washed with an equal volume of PBS. The EG cell colonies were then immersed in Shiff's Solution(Sigma, USA) at room temperature for 15 min. and then washed twice with an equal volume of PBS. The resulting EG cell colonies were observed under an inverted microscope. Chicken PGCs can be easily identified by the PAS reaction which stains glycogens in the cytoplasm (Meyer, D. B., Dev. Biol., 10, 154-190(1964)). As can be seen in Fig. 2, the EG cells after 4 passages can be stained by PAS, although the staining is relatively weak.

### (2) Anti-SSEA-1 antibody screening

The SSEA-1 epitope is characteristic of undifferentiated murine ES cells and has been as a criterion for distinguishing pluripotent stem cells (Solter, D. et al., Proc. Natl. Acad. Sci., 75, 5565-5596 (1978)). To determine whether the pluripotency of the EG cell possessed characteristic features of a pluripotent cell, the presence of SSEA-1 epitope were examined.

The EG cell colonies after 4 passages obtained in Example 2 were fixed to a plate in 1 % glutaraldehyde solution for 5 min. and washed twice with an equal volume of PBS. An ascites fluid of anti-SSEA-1 monoclonal antibody (MC-480; Solter, D. et al., Proc. Natl. Acad. Sci., 75, 5565-5596 (1978)) was purchased from the Development Studies Hybridoma Bank, Iowa University U.S.A., diluted 1,000 fold with PBS, and added to the EG cell colonies. The resulting EG cell colonies were reacted with avidin/biotin-conjugated alkaline phosphatase (Vector Lab., USA) and then with BCIP/NBT alkaline phosphatase substrate (BCIP/NBT alkaline phosphatase substrate kit IV, Vector Lab., USA). The reaction was stopped by adding 10 mM EDTA(pH 8.0) thereto.

As can be seen in Fig. 3, all EG cells after 4 passages are positive toward SSEA-1 staining showing that the SSEA-1 epitope is expressed by the chicken EG cells.

### (3) Proliferation assay

The EG cell colonies after 8 passages obtained in Example 2 were kept in solution containing bromodeoxyuridine (BrdU, a thymidine analog), at 37 °C for 1 hour and washed twice with an equal volume of PBS. The resulting EG cell colonies were stained by cell proliferation assay kit (Amersham, UK) and then counterstained with PAS by repeating the procedure of (1). EG cell colonies containing incorporated BrdU were detected using any anti-BrdU monoclonal antibody and a peroxidase/DAB system (Amersham, UK) .

Fig. 4 presents the proliferation assay result of EG cell colonies stained after 8 passages. As can be seen from Fig. 4, the EG cells after 8 passages are in continuous proliferation.

### (4) Alkaline phosphatase activity assay

The alkaline phosphatase activity of the EG cell colonies after 4 passages obtained in Example 2 was measured using alkaline phosphatase substrate kit IV(Vector Lab., USA) .

The result shows that the EG cells have scarcely alkaline phosphatase activity and did not regain the activity during subculture.

According to Swartz, W. J., Anat. Rec., 202, 379-385 (1982), alkaline phosphatase activity of avian PGCs is observably as early as 2 days of incubation but not after the entry of PGCs into genital ridge, which would suggest that the alkaline phosphatase activity of gonadal PGCs as well as EG cells passaged therefrom would indeed be very weak.

### (5) In vitro differentiation and immunohistochemical analysis

To examine whether embryoid bodies could formed from the chicken EG cells, the EG cell colonies after 4 passages obtained in Example 2 were gently agitated and centrifuged to obtain individually separated EG cells. The EG cells were suspended in EG cell culture media free from mLIF and then placed in a non-adhesive bacteriological petri dish. The media was changed every other day for 8 days and the morphology of the cells was monitored daily.

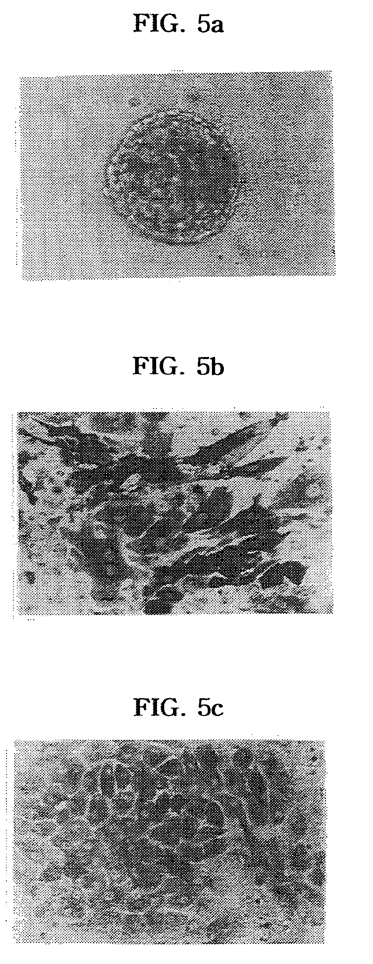

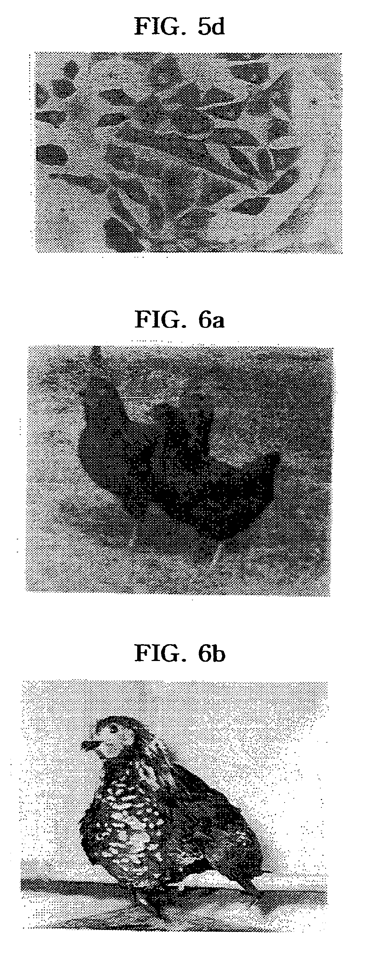

Fig. 5a displays the embryoid bodies formed from chicken EG cells in suspension after 8 days.

The embryoid bodies thus formed were collected and then distributed into a 96-well plate further to attach thereto and differentiate. The resulting cells were subjected to immunohistochemical analysis using antibodies for muscle-specific actin (Dako, USA), endoderm-specific α-1-fectoprotein (Dako, USA) and ectoderm-specific S100 (Dako, USA), together with an DAKO LSAB kit and avidin/biotin-conjugated peroxidase system(Dako, USA).

Figs. 5b to 5d illustrate the results of immunohistochemical analyses of the embryoid bodies using antibodies against actin, α-1-fectoprotein and S100, respectively. As can be seen from Figs. 5b to 5d, the EG cells are capable of differentiating in vitro into a variety of cell types, e.g., endoderm, mesoderm and ectoderm lineage.

### Example 4: Production of Chimeric Chicken

Korean Ogol chicken embryos at stage X or 13 to 17 (Eyal-Giladi, H. et al., Dev Biol, 49, 321-337(1976)) were used as recipients. The lateral part or pointed end of each Korean Ogol chicken egg was punctured to provide a small window and then the shell membrane was removed.

The EG cells after 3 or 4 passages obtained in Example 2 were suspended in EG cell culture media at a concentration of 10³ cells/µl and then 2 µl of the suspension was injected into the germinal cavity or blood vessel of the egg using a micropipette. Eggs injected with CEF were used as a control. The window of each egg was sealed twice with paraffin film, and then, the eggs were laid down with the pointed end at the bottom until hatching. The hatched chicks were allowed to grow for 3 months to obtain somatic chimera chickens.

Of 45 eggs with manipulated embryos, 8 eggs were hatched and, among the eight, 3 chicks (37.5 %) were externally chimeric. Among chimeric chicks, two white patched chicks were hatched from the embryos injected with the EG cells of 3 passages and one was from the embryo injected with the EG cells of 4 passages. The extent of white patching varied significantly among the 3 chicks. No white feather was observed with the control chick.

Fig. 6a exhibit, a pair of typical Korean Ogol chicken having black feathers; and Fig. 6b, the somatic chimera chicken having white patches around the neck and on the breast. The feather color of a White Leghorn is white due to the dominant pigmentation inhibitory gene(I/I), and a Korean Ogol chicken, black due to the recessive pigment gene(i/i). Accordingly, the above result suggests that the EG cells of the White Leghorn are capable of differentiating in vivo in the recipient Korean Ogol chicken embryo and, therefore, the EG cells are pluripotent in vivo. To verify the somatic chimerism of the chicken, genomic DNA samples were obtained by phenol extraction from the muscle, heart, liver and gonad of 5 chicks which died during hatching and then subjected to PCR analysis using White Leghorn-specific SCAR (sequence characterized amplified region) primers having nucleotide sequences of SEQ ID NO: 1 (forward primer) and SEQ ID NO: 2 (reverse primer).

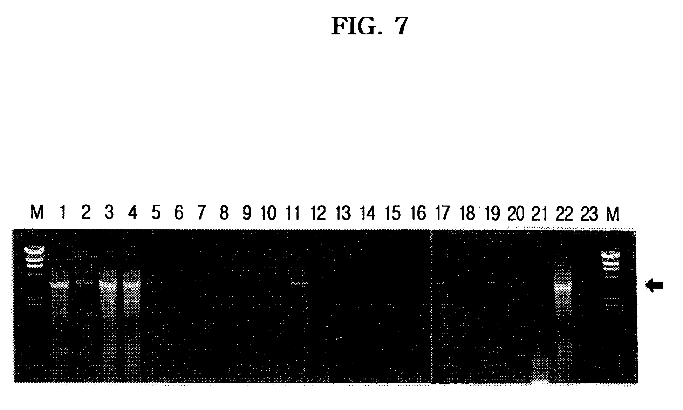

PCR reactions were carried out at a 25 µl scale using 50 to 100 ng of genomic DNA, 0.2 mM of each dNTP, 10 mM KCl, 1.5 mM MgCl₂, 0.4 pmol of forward primer, 0.4 pmol of reverse primer and 1 unit of Taq polymerase. The PCR program consisted of 1 minute of denaturation at 94 °C, 1 minute of annealing at 60 °C and 2 minutes of extension at 72 °C for 45 cycles in a DNA thermocycler (Perkin Elmer Cetus). The White Leghorn specific DNA fragment produced was approximately 3 kb. Fig. 7 depicts the results of PCR analysis for somatic chimerism: Lanes 1, 5, 9, 13 and 17, PCR products using the liver DNA; Lanes 2, 6, 10, 14 and 18, the muscle DNA; Lanes 3, 7, 11, 15 and 19, the heart DNA; Lanes 4, 8, 12, 16 and 20, the gonad DNA; Lane 21, Korean Ogol chicken genomic DNA; Lane 22, White Leghorn genomic DNA; and Lane 23, no template. As can be seen from Fig. 7, the somatic chimerisms were different between individuals: one of the 5 chicks showed somatic chimerism in all tissue samples (liver, heart, muscle, and gonads). The injected EG cells contributed to the gonads of two hatched chicks and the heart in all of the chicks; two chicks showed the somatic chimerism only in the heart. These results indicate that chicken EG cells can differentiate and contribute in vivo to various tissues including the gonads.

### Example 5: Transfection of Foreign Gene into PGCs or EG cells and Selection Thereof

A reporter gene (GFP or Lac Z) was transfected into PGCs or EG cells using electroporation and liposome, respectively. Transfection efficiency of the gene was about 80% in case of using electroporation and 30% in case of using liposome. Transfected PGCs or EG cells were passaged in a DMEM medium containing 350 µg/ml of neomycin to select the stably transfected PGCs or EG cells.

While the invention has been described with respect to the above specific embodiments, it should be recognized that various modifications and changes may be made to the invention by those skilled in the art which also fall within the scope of the invention as defined by the appended claims.

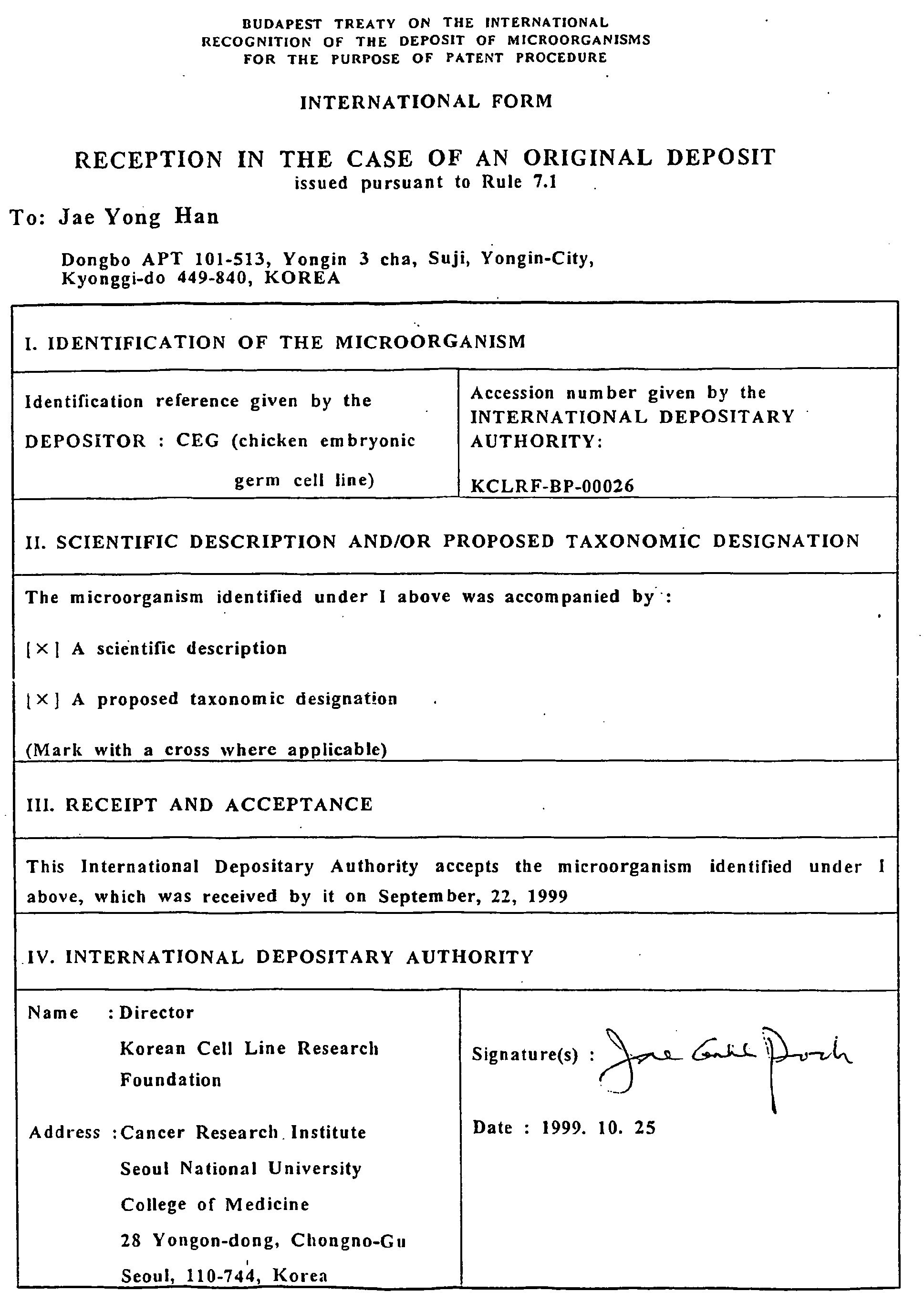
BUDAPEST TREATY ON THE INTERNATIONAL
RECOGNITION OF THE DEPOSIT OF MICROORGANISMS
FOR THE PURPOSE OF PATENT PROCEDURE

INTERNATIONAL FORM

RECEPTION IN THE CASE OF AN ORIGINAL DEPOSIT
issued pursuant to Rule 7.1

To: Jae Yong Han

Dongbo APT 101-513, Yongin 3 cha, Suji, Yongin-City,
Kyonggi-do 449-840, KOREA

I. IDENTIFICATION OF THE MICROORGANISM
Identification reference given by the
DEPOSITOR : CEG (chicken embryonic
germ cell line) | Accession number given by the
INTERNATIONAL DEPOSITARY
AUTHORITY:

KCLRF-BP-00026
II. SCIENTIFIC DESCRIPTION AND/OR PROPOSED TAXONOMIC DESIGNATION
The microorganism identified under I above was accompanied by :

[×] A scientific description

[×] A proposed taxonomic designation

(Mark with a cross where applicable)
III. RECEIPT AND ACCEPTANCE
This International Depositary Authority accepts the microorganism identified under I
above, which was received by it on September, 22, 1999
IV. INTERNATIONAL DEPOSITARY AUTHORITY
Name : Director
Korean Cell Line Research
Foundation

Address :Cancer Research Institute
Seoul National University
College of Medicine
28 Yongon-dong, Chongno-Gu
Seoul, 110-744, Korea | Signature(s) :

Date : 1999. 10. 25

## Claims

1. A process for preparing an established avian embryonic germ (EG) cell line comprising the steps of:
(a) culturing primordial germ cells (PGCs) isolated from an avian embryonic gonad in a medium supplemented with a cell growth factor and a differentiation inhibitory factor to obtain EG cell colonies;
(b) culturing the EG cells in the same medium as in step (a) by employing a feeder layer until the EG cells are colonized; and
(c) recovering and subculturing the EG cells in the same medium as in step (a) to establish the EG cell line.

2. The process of claim 1, wherein the avian embryonic gonad is at a stage ranging from 14 to 36.

3. The process of claim 2, wherein the avian embryonic gonad is at a stage ranging from 24 to 30.

4. The process of claim 1, wherein the avian species is turkey, chicken, quail, pheasant or duck.

5. The process of claim 1, wherein a layer of germinal ridge stroma cells(GRSCs) is employed as a feeder layer when culturing primordial germ cells in step (a).

6. The process of claim 1, wherein the growth factor is selected from the group consisting of stem cell factor (SCF), basic fibroblast growth factor (bFGF), interleukin-11 (IL-11), insulin-like growth factor-I (IGF-I) and a mixture thereof.

7. The process of claim 1, wherein the medium is supplemented with a growth factor selected from the group consisting of 0.05 to 500 ng/ml of SCF, 0.1 to 1000 ng/ml of bFGF, 0.0004 to 4 ng/ml of IL-11, 0.1 to 1000 ng/ml of IGF-I and a mixture thereof.

8. The process of claim 1, wherein the differentiation inhibitory factor is leukemia inhibitory factor (LIF).

9. The process of claim 8, wherein the amount of LIF is 0.1 to 1000 units/ml.

10. The process of claim 1, wherein the medium further comprises mammalian or avian serum.

11. The process of claim 1, wherein the medium further comprises a supplementary ingredient selected from the group consisting of sodium pyruvate, glutamine, β-mercaptoethanol and a mixture thereof.

12. The process of claim 1, wherein the feeder layer is mitotically active.

13. The process of claim 1 or 12, wherein the feeder layer is fibroblast or an equivalent thereof.

14. The process of claim 13, wherein the fibroblast is avian fibroblast or avian embryonic fibroblast.

15. The process of claim 14 , wherein the avian species is chicken.

16. An avian embryonic germ (EG) cell line prepared in accordance with the process of claim 1.

17. The avian EG cell line of claim 16, which can be maintained by repeated subculture.

18. The avian EG cell line of claim 16, which expresses SSEA-1 antigen, forms an embryoid body, and differentiates and contributes to various tissues.

19. The avian EG cell line of claim 16, which is a chicken embryonic germ cell line having characteristics substantially identical to that deposited under the accession number of KCLRF-BP-00026.

20. A process for preparing a somatic or germline chimera comprising injecting the avian EG cell of claim 16 into a non-human egg.

21. The process of claim 20, wherein the EG cell is injected into a germinal cavity or blood vessel of the non-human egg.

22. The process of claim 21, wherein the EG cell is injected into the germinal cavity of the non-human egg at a stage X.

23. The process of claim 21, wherein the EG cell is injected into the blood vessel of the non-human egg at a stage ranging from 13 to 17.

24. A process for transfecting a foreign gene into avian EG cells **characterized by** using electroporation or liposome.

25. A process for selecting stably transfected avain EG cells comprising passaging EG cells transfected by a foreign gene in a medium containing an antibiotic.

## Patentansprüche

1. Verfahren zur Herstellung einer etablierten avianen embryonalen Keimzelllinie (EG), wobei das Verfahren folgende Schritte umfasst:
(a) Kultivieren von primordialen Keimzellen (PGCs), die aus einer avianen embryonalen Gonade isoliert wurden, in einem Medium, das mit einem Zellwachstumsfaktor und einem differenzierungshemmenden Faktor angereichert ist, um EG-Zellkolonien zu erhalten;
(b) Kultivieren der EG-Zellen im gleichen Medium wie in Schritt (a) unter Verwendung einer Feeder-Schicht, bis die EG-Zellen Kolonien gebildet haben; und
(c) Gewinnung und Subkultivierung der EG-Zellen im gleichen Medium wie in Schritt (a) zur Etablierung der EG-Zelllinie.

2. Verfahren nach Anspruch 1, wobei sich die aviane embryonale Gonade in einem Stadium im Bereich von 14 bis 36 befindet.

3. Verfahren nach Anspruch 2, wobei sich die aviane embryonale Gonade in einem Stadium im Bereich von 24 bis 30 befindet.

4. Verfahren nach Anspruch 1, wobei es sich bei der avianen Spezies um Truthahn, Huhn, Wachtel, Fasan oder Ente handelt.

5. Verfahren nach Anspruch 1, wobei eine Schicht aus Keimleisten-Stromazellen (GRSCs) als Feeder-Schicht verwendet wird, wenn primordiale Keimzellen in Schritt (a) kultiviert werden.

6. Verfahren nach Anspruch 1, wobei der Wachstumsfaktor von der aus Stammzellenfaktor (SCF), basischem Fibroblastenwachstumsfaktor (bFGF), Interleukin-11 (IL-11), insulinartigem Wachstumsfaktor I (IGF-I) und einem Gemisch davon bestehenden Gruppe ausgewählt wird.

7. Verfahren nach Anspruch 1, wobei das Medium mit einem Wachstumsfaktor angereichert wird, der von der aus 0,05 bis 500 ng/ml SCF, 0,1 bis 1000 ng/ml bFGF, 0,0004 bis 4 ng/ml IL-11, 0,1 bis 1000 ng/ml IGF-I und einem Gemisch davon bestehenden Gruppe ausgewählt wird.

8. Verfahren nach Anspruch 1, wobei der differenzierungshemmende Faktor ein Leukämie-hemmender Faktor (LIF) ist.

9. Verfahren nach Anspruch 8, wobei die Menge an LIF 0,1 bis 1000 Einheiten/ml beträgt.

10. Verfahren nach Anspruch 1, wobei das Medium weiterhin Säugetier- oder avianes Serum umfasst.

11. Verfahren nach Anspruch 1, wobei das Medium weiterhin einen zusätzlichen Bestandteil umfasst, der von der aus Natriumpyruvat, Glutamin, β-Mercaptoethanol und einem Gemisch davon bestehenden Gruppe ausgewählt wird.

12. Verfahren nach Anspruch 1, wobei die Feeder-Schicht mitotisch aktiv ist.

13. Verfahren nach Anspruch 1 oder 12, wobei es sich bei der Feeder-Schicht um Fibroblasten oder ein Äquivalent dazu handelt.

14. Verfahren nach Anspruch 13, wobei es sich bei den Fibroblasten um aviane Fibroblasten oder aviane embryonale Fibroblasten handelt.

15. Verfahren nach Anspruch 14, wobei die aviane Spezies Huhn ist.

16. Aviane embryonale Keimzelllinie (EG), hergestellt gemäß dem Verfahren nach Anspruch 1.

17. Aviane EG-Zelllinie nach Anspruch 16, die durch wiederholtes Subkultivieren aufrechterhalten werden kann.

18. Aviane EG-Zelllinie nach Anspruch 16, die das Antigen SSEA-1 exprimiert, einen Embryoidkörper bildet und zu verschiedenen Geweben differenziert und beiträgt.

19. Aviane EG-Zelllinie nach Anspruch 16, bei der es sich um eine embryonale Keimzelllinie vom Huhn handelt und die Eigenschaften aufweist, die im Wesentlichen identisch zu der unter der Zugangsnummer KCLRF-BP-00026 hinterlegten Zelllinie sind.

20. Verfahren zur Herstellung einer somatischen oder Keimbahn-Chimäre, das das Injizieren der avianen EG-Zelllinie nach Anspruch 16 in ein nichtmenschliches Ei umfasst.

21. Verfahren nach Anspruch 20, wobei die EG-Zelle in eine Keimhöhle oder in ein Blutgefäß des nichtmenschlichen Eis injiziert wird.

22. Verfahren nach Anspruch 21, wobei die EG-Zelle in die Keimhöhle des nichtmenschlichen Eis in einem Stadium X injiziert wird.

23. Verfahren nach Anspruch 21, wobei die EG-Zelle in das Blutgefäß des nichtmenschlichen Eis in einem Stadium im Bereich von 13 bis 17 injiziert wird.

24. Verfahren zur Transfektion eines fremden Gens in aviane EG-Zellen, **gekennzeichnet dadurch, dass** E-lektroporation oder Liposom verwendet wird.

25. Verfahren zur Selektion stabil transfizierter avianer EG-Zellen, das das Passagieren von EG-Zellen umfasst, die mit einem fremden Gen transfiziert sind, in einem Medium, das ein Antibiotikum enthält.

## Revendications

1. Procédé pour préparer une lignée de cellules germinales embryonnaires (EG) aviaires établies comprenant les étapes de:
(a) culture de cellules germinales primordiales (PGCs) isolées à partir d'une gonade embryonnaire aviaire dans un milieu supplémenté en facteur de croissance cellulaire et en facteur inhibiteur de différentiation pour obtenir des colonies de cellules EG;
(b) culture de cellules EG dans le même milieu qu'à l'étape (a) en utilisant une couche nourricière jusqu'à ce que les cellules soient colonisées;
(c) récupération et sous-culture des cellules EG dans le même milieu qu'à l'étape (a) pour établir la lignée de cellules EG.

2. Procédé de la revendication 1, dans lequel la gonade embryonnaire aviaire est à un stade allant de 14 à 36.

3. Procédé de la revendication 2, dans lequel la gonade embryonnaire aviaire est à un stade allant de 24 à 30.

4. Procédé de la revendication 1, dans lequel l'espèce aviaire est la dinde, le poulet, la caille, le faisan ou le canard.

5. Procédé de la revendication 1, dans lequel une couche de cellules du stroma de la crête germinale (GRSCs) est utilisée comme couche nourricière pour la culture de cellules germinales primordiales à l'étape (a).

6. Procédé de la revendication 1, dans lequel le facteur de croissance est sélectionné dans le groupe constitué par le facteur de cellules souches (SCF), le facteur de croissance basique fibroblasique (bFGF), l'interleukine -11 (IL-11), le facteur de croissance de type I analogue à l'insuline (IGF-I) et un mélange ce ceux-ci.

7. Procédé de la revendication 1, dans lequel le milieu est supplémenté en facteur de croissance sélectionné dans le groupe constitué par 0.05 à 500 ng/ml de SCF, 0. à 1000 ng/ml de bFGF, 0.0004 à 4 ng/ml de IL-11, 0.1 à 1000 ng/ml de IGF-I et un mélange de ceux-ci.

8. Procédé de la revendication 1, dans lequel le facteur inhibiteur de différentiation est le facteur inhibiteur de la leucémie (LIF).

9. Procédé de la revendication 8, dans lequel la quantité de LIF est de 0,1 à 1000 unités/ml.

10. Procédé de la revendication 1, dans lequel le milieu comprend en outre du sérum mammalien ou aviaire.

11. Procédé de la revendication 1, dans lequel le milieu comprend en outre un ingrédient supplémentaire sélectionné dans le groupe constitué du pyruvate de sodium, de la glutamine, du bétamercaptoéthanol ou d'un mélange de ceux-ci.

12. Procédé de la revendication 1, dans lequel la couche nourricière est mitotiquement active.

13. Procédé de la revendication 1 ou 12, dans lequel la couche nourricière est du fibroblaste ou un équivalent.

14. Procédé de la revendication 13, dans lequel le fibroblaste est du fibroblaste aviaire ou du fibroblaste embryonnaire aviaire.

15. Procédé de la revendication 14, dans lequel l'espèce aviaire est le poulet ;

16. Lignée de cellules germinales embryonnaires (EG) préparée selon le procédé de la revendication 1.

17. Lignée de cellules EG de la revendication 16, qui peut être conservée par sous-culture répétée.

18. Lignée de cellules EG de la revendication 16, qui exprime l'antigène SSEA-1, forme un corps embryoïde, se différencie et contribue à plusieurs tissus.

19. Lignée de cellules EG de la revendication 16, qui est une lignée de cellules germinales embryonnaires de poulet ayant des caractéristiques sensiblement identiques à celles déposées sous le numéro d'accession KCLRF-BP-00026.

20. Procédé pour fabriquer des chimères de lignée germinale ou somatique comprenant l'injection de la cellule EG aviaire de la revendication 16 dans un oeuf non humain.

21. Procédé de la revendication 20, dans lequel la cellule EG est injectée dans la cavité germinale ou dans un vaisseau sanguin de l'oeuf non humain.

22. Procédé de la revendication 21, dans lequel la cellule EG est injectée dans la cavité germinale d'un oeuf non humain à une phase X.

23. Procédé de la revendication 21 dans lequel la cellule EG est injectée dans le vaisseau sanguin de l'oeuf non humain à une phase allant de 13 à 17.

24. Procédé pour transfecter un gène étranger dans une cellule EG aviaire **caractérisé par** l'utilisation d'électroporation ou de liposome.

25. Procédé pour sélectionner des cellules EG aviaires stablement transfectées comprenant le passage de cellules EG transfectées par un gène étranger dans un milieu contenant un antibiotique.


### SEQUENCE LISTING

<110> HAN, JAE YONG
   HAM MI PHARM. CO., LTD.
   PARK, TAE SUB
<120> AVIAN PLURIPOTENT EMBRYONIC GERM CELL LINE
<130> PC91143/HMY
<150> KR 1999-4860
   <151> 1999-02-11
<160> 2
<170> KOPATIN 1.5
<210> 1
   <211> 24
   <212> DNA
   <213> Artificial Sequence
<220>
   <223> White Leghorn-specific SCAR primer(forward primer)
<400> 1
   aacgcgtaga gttgcaggga tcag 24
<210> 2
   <211> 24
   <212> DNA
   <213> Artificial Sequence
<220>
   <223> White Leghorn-specific SCAR primer (reverse primer)
<400> 2
   aacgcgtaga tattcgagta cctt 24